# EUROPEAN PATENT SPECIFICATION

(11) **EP 4 574 124 B1**
(45) Date of publication and mention of the grant of the patent: **07.10.2026**
(21) Application number: 24218593.2
(22) Date of filing: 10.12.2024
(51) Int. Cl.: A61G 7/018, A61G 7/05, G16H 40/20

(54) **INTRA-ROOM BED MOVEMENT MONITORING FOR WIRELESSLY CONNECTED BED**
BETTBEWEGUNGSÜBERWACHUNG IM RAUM FÜR DRAHTLOS VERBUNDENES BETT
SURVEILLANCE DE MOUVEMENT DE LIT INTRA-SALLE POUR LIT CONNECTÉ SANS FIL

(30) Priority: 18.12.2023 US 202363611360 P
(43) Date of publication of application: 25.06.2025
(60) Divisional application: 26201477.2
(73) Proprietor: Hill-Rom Services, Inc., Batesville, IN 47006-9167 (US)
(72) Inventor: APPRIOU, Ronan, Batesville, 47006-9167 (US); SCOLAN, Maxime E, Batesville, 47006-9167 (US)
(74) Representative: Reddie & Grose LLP

(56) References cited:
- US-A1- 2011 162 141
- US-A1- 2019 358 997
- US-A1- 2020 268 579
- US-A1- 2023 277 397

## Description

The present disclosure relates to wirelessly connected devices, and more particularly to intra-room device movement monitoring for wirelessly connected devices.

In many healthcare facility environments, medical devices, for example patient support apparatuses, can connect to a local wireless network within the healthcare facility. Generally, a connected device needs to be located in, and associated with, a room in the healthcare facility to properly transmit data to and from the local wireless network. Typically, a healthcare facility uses manual locating to locate the device in a room because manual location is cheaper than using a real-time locating system. That is, a caregiver selects, from a user interface of the device, the correct room that the device is located in from a list of possible available rooms. However, during a power down of the device, for example, unplugging the device for room cleaning, the caregivers have no way of knowing whether the device remained in the associated room or if the device was moved to another room. To avoid any risk of the device being moved, devices are allowed to automatically relocate to the previous room only within a minute. In the case of a longer power off, for example, the device being disconnected from a main power source to clean the room, the caregiver is required to manually relocate the device, even if the device has not been moved. Accordingly, in some cases, the device is physically in the same room, but the caregivers are still required to specify the same location each time the device is powered down.

US 2020/268579 A1 discloses patient beds having equipment used in connection with determining locations of the patient beds in a healthcare facility.

The invention is set out in the appended set of claims.

The present disclosure includes one or more of the following features alone or in any combination.

According to a first aspect of the disclosed embodiments, a control system for a device that is positioned in and associated with a room of a facility is provided. The control system includes a brake switch to determine whether a brake of the device is active. A caster swivel sensor determines an angular orientation (β) of a wheel of the device about a caster swivel axis of a wheel assembly of the device. A wheel rotation sensor determines a distance that the wheel of the device has moved. At least one magnet is coupled to the wheel of the device to activate the wheel rotation sensor. The control system wakes up when at least one of the brake is deactivated and the device is unplugged. In response to the device waking up, the caster swivel sensor determines the angular orientation (β) of the wheel of the device and the wheel rotation sensor detects movement of the wheel based on detection of the at least one magnet. In response to the brake being reactivated, the control system determines an end distance that the device has moved based on the detected angular orientation (β) of the wheel and the detected movement of the wheel. If the device has moved less than a predetermined distance, the control system automatically reassociates the device to the same room.

In some embodiments of the first aspect, if the device has moved more than the predetermined distance, the control system may automatically notify a user to associate a new room to the device. The predetermined distance may be one meter.

Optionally, in the first aspect, the caster swivel sensor may determine the angular orientation (β) of the wheel relative to a preset position of the wheel. The wheel may be attached to a base that rotates relative to a post coupled to a frame of the device. In the preset position, a longitudinal axis of the base may be parallel to a side of the frame of the device. A coefficient (Coeff) of the wheel rotation sensor may be equal to (π times a diameter of the wheel)/(a number of magnets on the wheel). An end distance (D) that the device has moved may equal sqr(X²+Y²), wherein Xₙ=Xₙ₋₁(1+ Coeff cos(β)) and Yₙ=Yₙ₋₁(1+ Coeff sin(β)), wherein X is a distance in the x direction and Y is a distance in the y direction.

It may be desired, in the first aspect, that the control system may enter a sleep mode if movement of the wheel is not detected for a predetermined period of time. The predetermined period of time may be one minute. After returning to the sleep mode, the control system may wake up in response to the wheel rotation sensor detecting movement of the wheel. The control system may enter a sleep mode, after the device is automatically reassociated to the same room.

In some embodiments of the first aspect, the device may be reassociated to the room of the facility to connect the device to a wireless network in the room. The facility may include a healthcare facility and the device may include a medical device. The medical device may be a patient support apparatus.

Optionally, in the first aspect, the wheel may be attached to a base that rotates relative to a post coupled to a frame of the device. The caster swivel sensor may be attached to the base. The wheel rotation sensor may be coupled to the base. The at least one magnet may include a plurality of magnets. The plurality of magnets may be equally circumferentially spaced around the wheel. The caster swivel sensor may include an accelerometer. The wheel rotation sensor may include a Hall effect sensor.

According to a second aspect of the disclosed embodiments, a method for automatically reassociating a room in a facility to a device that is moved from a position in the room is provided, wherein the device is associated to the room of the facility prior to being moved. The method includes waking up a control system when at least one of a brake of the device is deactivated and the device is unplugged. The method also includes, in response to the device waking up, determining, with an accelerometer, an angular orientation (β) of a wheel of the device about a caster swivel axis of a wheel assembly of the device and detecting, with a Hall effect sensor, movement of the wheel based on detection of at least one magnet coupled to the wheel. The method also includes, in response to the brake being reactivated, determining an end distance that the device has moved based on the detected angular orientation (β) of the wheel and the detected movement of the wheel. If the device has moved less than a predetermined distance, the method also includes automatically reassociating the device to the same room.

In some embodiments of the second aspect, the method may also include automatically notifying a user to associate a new room to the device, if the device has moved more than the predetermined distance. The predetermined distance may be one meter.\

Optionally, in the second aspect, the method may also include determining, with the accelerometer, the angular orientation (β) of the wheel relative to a preset position of the wheel. The wheel may be attached to a base that rotates relative to a post coupled to a frame of the device. In the preset position, a longitudinal axis of the base may be parallel to a side of the frame of the device. A coefficient (Coeff) of the Hall effect sensor may be equal to (π times a diameter of the wheel)/(a number of magnets on the wheel). An end distance (D) that the device has moved may equal sqr(X²+Y²), wherein Xₙ=Xₙ₋₁(1+ Coeff cos(β)) and Yₙ=Yₙ₋₁(1+ Coeff sin(β)), wherein X is a distance in the x direction and Y is a distance in the y direction.

It may be desired, in the second aspect, that method also includes activating a sleep mode if movement of the wheel is not detected for a predetermined period of time. The predetermined period of time may be one minute. The method may also include waking up the control system, after returning to the sleep mode, in response to the Hall effect sensor detecting movement of the wheel. The control system may enter a sleep mode, after the device is automatically reassociated to the same room.

In some embodiments of the second aspect, the method may also include reassociating the device to the room of the facility to connect the device to a wireless network in the room. The facility may include a healthcare facility and the device may include a medical device. The medical device may be a patient support apparatus.

Optionally, in the second aspect, the wheel may be attached to a base that rotates relative to a post coupled to a frame of the device. The accelerometer may be attached to the base. The Hall effect sensor may be coupled to the base. The at least one magnet may include a plurality of magnets. The plurality of magnets may be equally circumferentially spaced around the wheel.

According to a third aspect of the disclosed embodiments, a control system for a patient support apparatus that is positioned in and associated with a room of a healthcare facility is provided. The control system includes an accelerometer to determine an angular orientation (β) of a wheel of the bed about a caster swivel axis of a wheel assembly of the bed. A Hall effect sensor determines a distance that the wheel of the bed has moved by detecting movement of at least one magnet coupled to the wheel. In response to the bed being moved, the control system determines an end distance that the bed has moved based on a detected angular orientation (β) of the wheel, as measured by the accelerometer, and movement of the wheel, as detected by the Hall effect sensor. If the bed has moved less than a predetermined distance, the bed is automatically reassociated to the same room to enable connection to a wireless network in the room.

Additional features, which alone or in combination with any other feature(s), such as those listed above and/or those listed in the claims, may comprise patentable subject matter and will become apparent to those skilled in the art upon consideration of the following detailed description of various embodiments exemplifying the best mode of carrying out the embodiments as presently perceived.

The invention will now be further described by way of example with reference to the accompanying drawings, in which:
FIG. 1 is a perspective view of a patient support apparatus in accordance with an embodiment;
FIG. 2 is a schematic view of a control system for the patient support apparatus shown in FIG. 1, wherein the control system automatically reassociates a room to the patient support apparatus after the patient support apparatus has been unplugged;
FIG. 3 is a side elevation view of a wheel assembly of the patient support apparatus shown in FIG. 1;
FIG. 4 is a side cut-away view of the wheel assembly of the patient support apparatus taken along the line 4-4 shown in FIG. 3, wherein a Hall effect sensor is coupled to a base of the wheel assembly, and a magnet disk having a plurality of magnets is coupled to a wheel of the wheel assembly;
FIG. 5 is a top view of the wheel assembly shown in FIG. 3 showing the Hall effect sensor coupled to the base and the magnet disk coupled to the wheel, wherein an accelerometer is also coupled to the base to measure an angular orientation (β) of a longitudinal axis of the base relative a longitudinal axis that is parallel to at least one frame member of the patient support apparatus, and wherein the longitudinal axis of the base is oriented 0 degrees relative the longitudinal axis that is parallel to the at least one frame member;
FIG. 6 is a top view of the wheel assembly similar to FIG. 5, wherein the longitudinal axis of the base is oriented greater than 0 degrees relative the longitudinal axis that is parallel to the at least one frame member; and
FIG. 7 is a flowchart for a method of automatically reassociating a room to the patient support apparatus after the patient support apparatus has been unplugged.

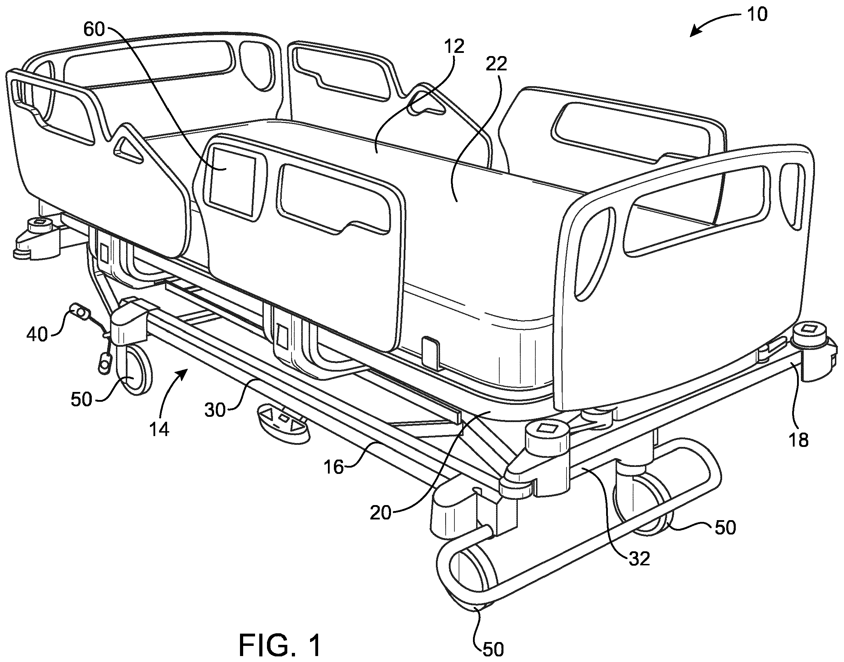

Referring to FIG. 1, a device 10 in accordance with an embodiment is configured for use in a facility. In an exemplary embodiment, the facility includes a healthcare facility and the device 10 includes a medical device. More specifically, in the exemplary embodiment the device 10 is a patient support apparatus 12, as illustrated in FIG. 1. The patient support apparatus 12 includes a base 14 having a base frame 16 connected to an intermediate frame 18. An articulated deck 20 is coupled to the intermediate frame 18. A mattress 22 is carried by the articulated deck 20 and provides a sleeping surface or support surface 24 configured to receive a patient (not shown). The base frame 16 includes a pair of parallel side frame sections 30 coupled by a pair of parallel end frame sections 32 that extend perpendicular to the parallel side frame sections 30. Wheel assemblies 50 extend downward from the side frame sections 30 and enable the patient support apparatus 12 to be rolled throughout the healthcare facility. A brake 40 is provided to lock the wheel assemblies 50 to prevent movement of the patient support apparatus 12.

The patient support apparatus 12 includes at least one graphical user interface (GUI) 60 that displays various screens to enable a caregiver to operate various functions of the patient support apparatus 12 by selecting user inputs adjacent to or provided on the GUI 60. For example, in some embodiments, movement of the articulated deck 20 is controlled via the GUI 60. In some embodiments, the patient support apparatus 12 is capable of being connected to a local network within the healthcare facility. For example, the patient support apparatus 12 is wirelessly connected to the local network, in some embodiments. To connect the patient support apparatus 12 to a local network, the patient support apparatus 12 is associated with a room in the healthcare facility. For example, in an embodiment, the caregiver associates the room to the patient support apparatus 12 using the GUI 60. The associated room enables the patient support apparatus 12 to be wirelessly connected to the correct local network in the healthcare facility. In some embodiments, the patient support apparatus 12 needs to be reassociated to a room in response to the patient support apparatus 12 being unplugged, or otherwise powered down, and plugged back in, or otherwise powered.

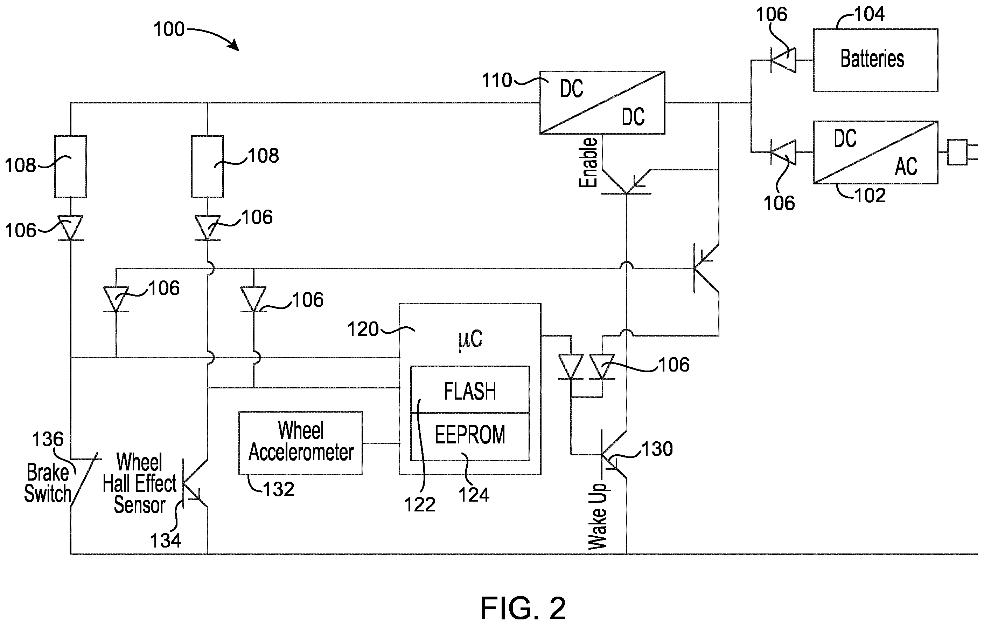

Referring to FIG. 2, a control system 100 of the patient support apparatus 12 is configured to automatically reassociate a room to the patient support apparatus 12 after the patient support apparatus 12 has been unplugged. The control system 100 includes a main AC/DC power source 102 that is configured to be plugged into an outlet of the healthcare facility. A battery 104 is provided to supply power to the patient support apparatus 12 in the event that the power source 102 is unplugged. A converter 110 converts the power in the control system 100 to direct current. The control system 100 includes a plurality of diodes 106 and resistors 108. In an exemplary embodiment, the resistors 108 are current limiting resistors. A microprocessor 120 is provided having a flash memory 122 and an electrically erasable programmable read only memory (EEPROM) 124. The flash memory 122 carries instructions that are carried out by the processor 120 to perform the steps described herein. The EEPROM 124 stores data collected by the components of the control system 100 described herein. In an embodiment, the microprocessor 120 includes a timer. In one embodiment, the timer begins counting from zero is response to the components of the control system 100 being activated.

] A wake up switch 130 detects when the patient support apparatus 12 is moving so that the control system 100 can begin tracking movement data related to the patient support apparatus 12. For example, an accelerometer 132 is provided to determine an angular orientation of a wheel (described below) of the wheel assembly 50 about a caster swivel axis of the wheel assembly 50 and a Hall effect sensor 134 is provided to detect rotation of the wheel. A brake switch 136 is further provided to detect whether the wheel assembly 50 is locked. In response to the brake switch 136 detecting that the wheel assembly 50 is unlocked, the control system 100 begins tracking movement of the patient support apparatus 12. Data related to the movement of the patient support apparatus 12 is stored in the EEPROM 124. In response to the brake switch 136 detecting that the wheel assembly 50 has been locked, the control system 100 determines an end distance that the patient support apparatus 12 traveled based on the data stored in the EEPROM 124.

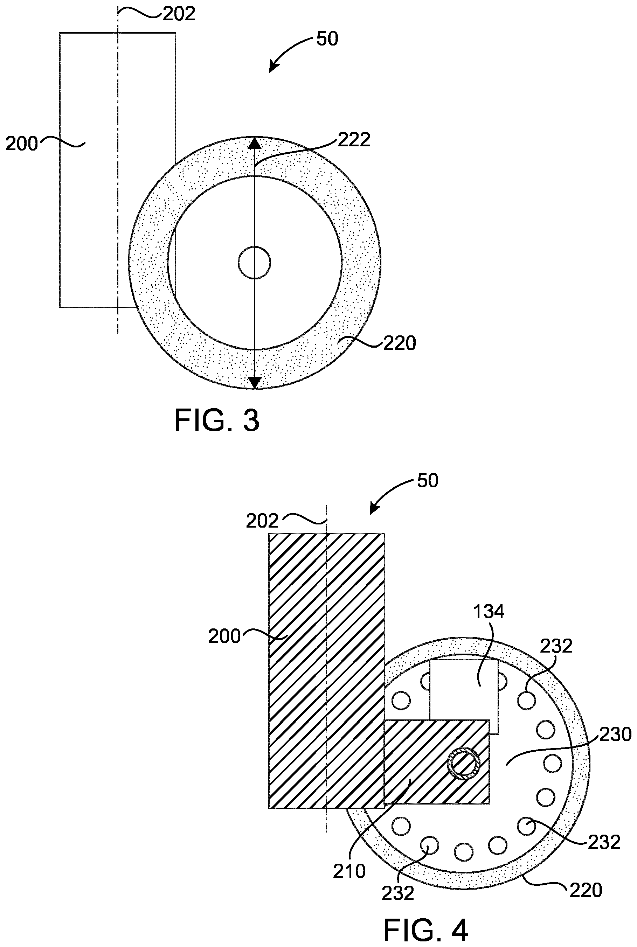

FIGS. 3-5 illustrate an exemplary wheel assembly 50. It will be appreciated that, in some embodiments, only one wheel assembly 50 of the patient support apparatus 12 is configured with the components described herein. Accordingly, the data processed by the control system 100 is acquired from only the one wheel assembly 50. In other embodiments, any number of wheel assemblies 50 of the patient support apparatus 12 include the components described herein. In such an embodiment, the control system 100 processes data from each wheel assembly 50 having the described components. The wheel assembly 50 includes a vertically extending post 200 that extends from the base frame 16 toward the floor along an axis 202. The wheel assembly 50 is configured to rotate about the axis 202. A base 210 extends horizontally from the post 200 along a base axis 212. In the exemplary embodiment, the base axis 212 extends substantially perpendicular to the axis 202. A pair of adjacent wheels 220 are attached to the base 210 and configured to rotated relative to the base 210 to roll the patient support apparatus 12. It will be appreciated that although the exemplary embodiment illustrates two wheels 220, the wheel assembly 50, in some embodiments, includes only one wheel 220. Each wheel 220 has a diameter 222.

The Hall effect sensor 134 is coupled to the base 210 of the wheel assembly 50. A magnet disk 230 is coupled to one of the wheels 220. The magnet disk 230 includes a plurality of magnets 232 attached thereto. It will be appreciated that the magnet disk 230 can include any number of magnets 232. The number of magnets 232 provided is factored into the calculations made by the control system 100, as described in more detail below. The magnets 232 are equally and circumferentially spaced around the magnet disk 230. The Hall effect sensor 134 detects each magnet 232 as the magnet 232 passes the Hall effect sensor 134 during rotation of the wheel 220 caused by movement of the patient support apparatus 12.

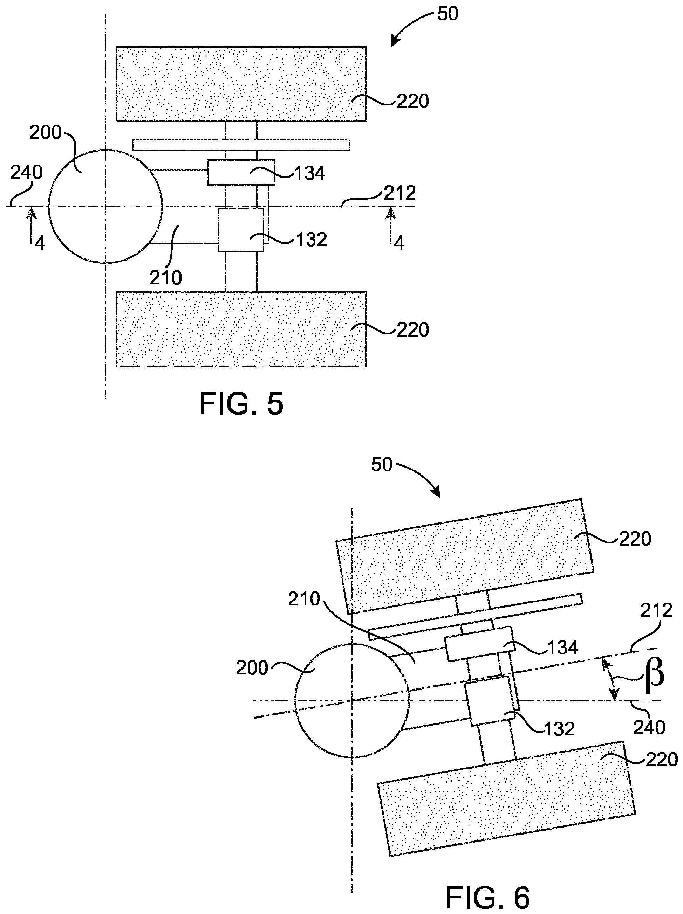

As illustrated in FIGs. 5-6, the accelerometer 132 is also coupled to the base 210 of the wheel assembly 50 to measure an angular orientation (β) of the wheel assembly 50 relative to a longitudinal axis 240 that is parallel to at least one frame member of the patient support apparatus 12. In one embodiment, the axis 240 is parallel to one of the side frame sections 30 of the base frame 16. In another embodiment, the axis 240 is parallel to one of end frame sections 32 of the base frame 16. In an exemplary embodiment, the accelerometer 132 measures the angular orientation (β) of the base axis 212 of the base 210 relative to the longitudinal axis 240. That is, as the wheel 220 and the base 210 rotate relative to the post 200, the accelerometer 132 measures a degree of the rotation. FIG. 5 illustrates the base 210 in a preset position, wherein the angular orientation (β) is zero degrees. FIG. 6 illustrates the base 210 at an angular orientation (β) that is greater than zero degrees relative to the preset position. As described in more detail below, the angular orientation (β) is a factor in determining an end distance that the patient support apparatus 12 has moved.

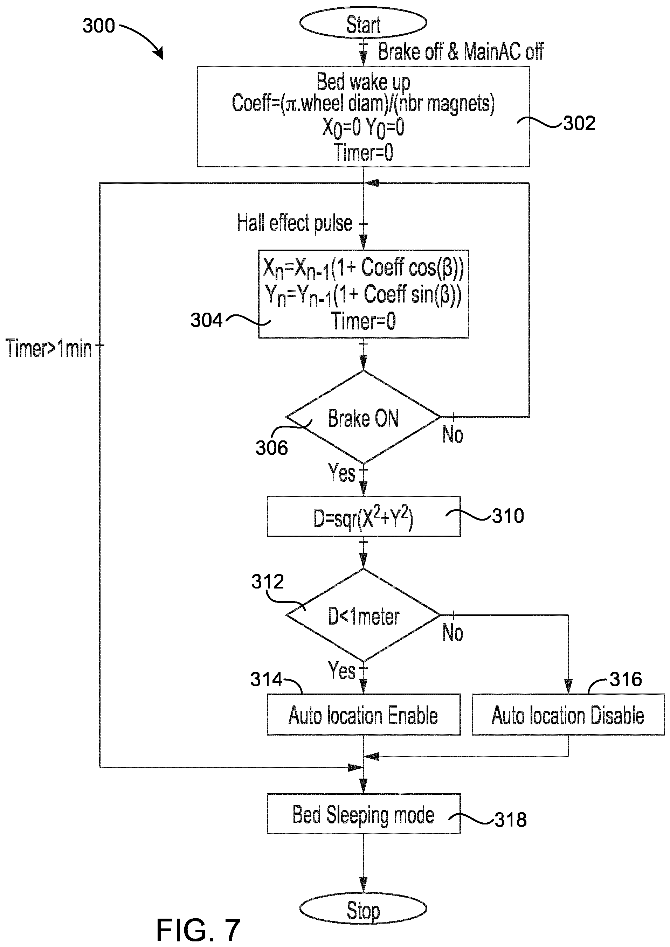

Referring to FIG. 7, a method 300 of automatically re associate a room to the patient support apparatus 12 after the patient support apparatus 12 has been unplugged starts in response to the main power source 102 being unplugged and the brake switch 136 detecting that the brake is deactivated and the wheel assembly 50 is unlocked thereby allowing the patient support apparatus 12 to be moved. In response, at block 302, the wake up switch 130 activates the control system 100. In response to the control system 100 being activated, the control system 100 determines a coefficient of the Hall effect sensor 134 using the equation, Coeff=(π.wheel diam)/(nbr magnets), wherein Coeff is the coefficient, wheel diam equals the diameter 222 of the wheel 220, and nbr magnets equals the number of magnets 232 on the magnet disk 230. Initially, when the timer is at zero, a distance moved in the x-direction (X) equals zero and a distance moved in the y-direction (Y) equals zero. Also, in response to the control system 100 waking up, the accelerometer 132 determines the angular orientation (β) of the wheel 220 and the Hall effect sensor 134 detects movement of the wheel 220 by generating pulses that detect movement of the magnets 232. In particular movement of the wheel 220 is detected by detecting an amount of rotation of the wheel 220 about a wheel rotation axis.

At block 304, the control system 100 calculates a distance moved in the X direction at various counts n using the equation Xₙ=Xₙ₋₁(1+ Coeff cos(β)), wherein X₀=0. The control system 100 also calculates a distance moved in the Y direction at various counts n using the equation Yₙ=Yₙ₋₁(1+ Coeff sin(β)), wherein Y₀=0. The control system 100 then determines at block 306, whether the brake switch 136 has been reactivated. If the brake has not been reactivated, the control system 100 continues to detect movement in the X and Y directions, at block 304.

If the brake has been reactivated, at 310, the control system 100 the calculates an end distance (D) that the patient support apparatus 12 has moved using the equation sqr(X²+Y²). Notably, if the patient support apparatus 12 is moved in a first direction away from an initial location and is then moved in an opposite direction back toward the initial location, the control system 100 calculates the final distance from the initial location as the end distance (D) because the X and Y values will become negative when moving back to the initial location. In response to determining the end distance (D), the control system 100 determines whether the end distance (D) is less than one meter, at block 312. If the end distance (D) is less than a predetermined distance, at block 314, the control system activates automatic location, thereby allowing the patient support apparatus 12 to be automatically reassociated to the initial room that the patient support apparatus 12 was located in when the power 102 was unplugged. Accordingly, the patient support apparatus 12 can be automatically connected to the local network corresponding to the room. If the end distance (D) is greater than the predetermined distance, at block 316, automatic locating is disabled, thereby requiring the caregiver to manually locate the patient support apparatus 12 to associate the apparatus 12 to the appropriate room in the healthcare facility. In an exemplary embodiment, the predetermined distance is one meter. At block 318, the control system 100 goes back into sleep mode after processing the method 300. In some embodiments, the control system 100 goes back into sleep mode after a predetermined period of time, for example, one minute.

## Claims

1. A control system (100) for a device (10) that is positioned in and associated with a room of a facility, the control system comprising:
a brake switch (136) to determine whether a brake of the device is active,
a caster swivel sensor to determine an angular orientation (β) of a wheel of the device about a caster swivel axis of a wheel assembly of the device,
a wheel rotation sensor to determine a distance that the wheel of the device has moved, and
at least one magnet coupled to the wheel of the device to activate the wheel rotation sensor,
wherein the control system wakes up when at least one of the brake is deactivated and the device is unplugged,
wherein, in response to the device waking up, the caster swivel sensor determines the angular orientation (β) of the wheel of the device and the wheel rotation sensor detects movement of the wheel based on detection of the at least one magnet,
wherein, in response to the brake being reactivated, the control system determines an end distance that the device has moved based on the detected angular orientation (β) of the wheel and the detected movement of the wheel, and
wherein, if the device has moved less than a predetermined distance, the control system automatically reassociates the device to the same room.

2. The control system of claim 1, wherein, if the device has moved more than the predetermined distance, the control system automatically notifies a user to associate a new room to the device, and wherein preferably the predetermined distance is one meter.

3. The control system of either claim 1 or claim 2, wherein the caster swivel sensor determines the angular orientation (β) of the wheel relative to a preset position of the wheel.

4. The control system of claim 3, wherein the wheel is attached to a base that rotates relative to a post coupled to a frame of the device, wherein, in the preset position, a longitudinal axis of the base is parallel to a side of the frame of the device.

5. The control system of claim 4, wherein a coefficient (Coeff) of the wheel rotation sensor is equal to (π times a diameter of the wheel)/(a number of magnets on the wheel).

6. The control system of claim 5, wherein an end distance (D) that the device has moved equals sqr(X²+Y²), wherein Xₙ=Xₙ₋₁(1+ Coeff cos(β)) and Yₙ=Yₙ₋₁(1+ Coeff sin(β)), wherein X is a distance in the x direction and Y is a distance in the y direction.

7. The control system of any preceding claim, wherein the control system enters a sleep mode if movement of the wheel is not detected for a predetermined period of time, and wherein preferably the predetermined period of time is one minute.

8. The control system of claim 7, wherein, after returning to the sleep mode, the control system wakes up in response to the wheel rotation sensor detecting movement of the wheel.

9. The control system of any preceding claim, wherein the control system enters a sleep mode, after the device is automatically reassociated to the same room.

10. The control system of any preceding claim, wherein the device is reassociated to the room of the facility to connect the device to a wireless network in the room.

11. The control system of any preceding claim, wherein the wheel is attached to a base that rotates relative to a post coupled to a frame of the device, wherein the caster swivel sensor is attached to the base.

12. The control system of claim 11, wherein the wheel rotation sensor is coupled to the base.

13. The control system of any preceding claim, wherein the at least one magnet includes a plurality of magnets.

14. The control system of claim 13, wherein the plurality of magnets are equally circumferentially spaced around the wheel.

15. The control system of any preceding claim, wherein the caster swivel sensor includes an accelerometer and/or the wheel rotation sensor includes a Hall effect sensor.

## Patentansprüche

1. Steuersystem (100) für eine Vorrichtung (10), die in einem Raum einer Einrichtung positioniert ist und diesem zugeordnet ist, wobei das Steuersystem Folgendes umfasst:
einen Bremsschalter (136) zum Bestimmen, ob eine Bremse der Vorrichtung aktiv ist,
einen Lenkrollenschwenksensor zum Bestimmen einer Winkelausrichtung (β) eines Rads der Vorrichtung um eine Lenkrollenschwenkachse einer Radanordnung der Vorrichtung,
einen Raddrehungssensor zum Bestimmen einer Entfernung, die das Rad der Vorrichtung zurückgelegt hat, und
mindestens einen Magnet, der zum Aktivieren des Raddrehungssensors mit dem Rad der Vorrichtung gekoppelt ist,
wobei das Steuersystem aufwacht, wenn die Bremse deaktiviert wird und/oder die Vorrichtung von der Stromversorgung getrennt wird,
wobei, als Reaktion auf das Aufwachen der Vorrichtung, der Lenkrollenschwenksensor die Winkelausrichtung (β) des Rads der Vorrichtung bestimmt und der Raddrehungssensor eine Bewegung des Rads auf Basis der Erkennung des mindestens einen Magneten erkennt,
wobei, als Reaktion auf das Wiederaktivieren der Bremse, das Steuersystem auf Basis der erkannten Winkelausrichtung (β) des Rads und der erkannten Bewegung des Rads eine abschließende Entfernung bestimmt, die die Vorrichtung zurückgelegt hat, und
wobei, wenn die Vorrichtung weniger als eine vorbestimmte Entfernung zurückgelegt hat, das Steuersystem die Vorrichtung automatisch wieder demselben Raum zuordnet.

2. Steuersystem nach Anspruch 1, wobei, wenn die Vorrichtung mehr als die vorbestimmte Entfernung zurückgelegt hat, das Steuersystem automatisch einen Benutzer benachrichtigt, um der Vorrichtung einen neuen Raum zuzuordnen, und wobei vorzugsweise die vorbestimmte Entfernung ein Meter ist.

3. Steuersystem nach Anspruch 1 oder Anspruch 2, wobei der Lenkrollenschwenksensor die Winkelausrichtung (β) des Rads relativ zu einer voreingestellten Stellung des Rades bestimmt.

4. Steuersystem nach Anspruch 3, wobei das Rad an einer Basis angebracht ist, die sich relativ zu einer mit einem Rahmen der Vorrichtung gekoppelten Stütze dreht, wobei, in der voreingestellten Stellung, eine Längsachse der Basis parallel zu einer Seite des Rahmens der Vorrichtung ist.

5. Steuersystem nach Anspruch 4, wobei ein Koeffizient (Coeff) des Raddrehungssensors gleich (π-Mal dem Durchmesser des Rads)/(Anzahl von Magneten an dem Rad) ist.

6. Steuersystem nach Anspruch 5, wobei eine abschließende Entfernung (D), die die Vorrichtung zurückgelegt hat, gleich sqr(X²+Y²) ist, wobei Xₙ=Xₙ₋₁(1+ Coeff cos(β)) und Yₙ=Yₙ₋₁(1+ Coeff sin(β)), wobei X eine Entfernung in der x-Richtung ist und Y eine Entfernung in der y-Richtung ist.

7. Steuersystem nach einem der vorhergehenden Ansprüche, wobei das Steuersystem in einen Schlafmodus übergeht, wenn für eine vorbestimmte Zeitspanne keine Bewegung des Rads erkannt wird, und wobei vorzugsweise die vorbestimmte Zeitspanne eine Minute beträgt.

8. Steuersystem nach Anspruch 7, wobei das Steuersystem nach dem Zurückkehren in den Schlafmodus als Reaktion darauf aufwacht, dass der Raddrehungssensor eine Bewegung des Rads erkennt.

9. Steuersystem nach einem der vorhergehenden Ansprüche, wobei das Steuersystem in einen Schlafmodus übergeht, nachdem die Vorrichtung automatisch wieder demselben Raum zugeordnet worden ist.

10. Steuersystem nach einem der vorhergehenden Ansprüche, wobei die Vorrichtung dem Raum der Einrichtung wieder zugeordnet wird, um die Vorrichtung mit einem drahtlosen Netzwerk in dem Raum zu verbinden.

11. Steuersystem nach einem der vorhergehenden Ansprüche, wobei das Rad an einer Basis angebracht ist, die sich relativ zu einer mit einem Rahmen der Vorrichtung gekoppelten Stütze dreht, wobei der Lenkrollenschwenksensor an der Basis angebracht ist.

12. Steuersystem nach Anspruch 11, wobei der Raddrehungssensor mit der Basis gekoppelt ist.

13. Steuersystem nach einem der vorhergehenden Ansprüche, wobei der mindestens eine Magnet mehrere Magneten beinhaltet.

14. Steuersystem nach Anspruch 13, wobei die mehreren Magneten gleichmäßig in Umfangsrichtung voneinander beabstandet um das Rad angeordnet sind.

15. Steuersystem nach einem der vorhergehenden Ansprüche, wobei der Lenkrollenschwenksensor einen Beschleunigungsmesser beinhaltet und/oder der Raddrehungssensor einen Halleffektsensor beinhaltet.

## Revendications

1. Système de commande (100) pour un dispositif (10) positionné dans une salle d'une installation et associé à celle-ci, le système de commande comprenant :
un commutateur de frein (136) pour déterminer qu'un frein du dispositif est actif ou non,
un capteur de pivotement de roulette pour déterminer une orientation angulaire (β) d'une roue du dispositif autour d'un axe de pivotement de roulette d'un ensemble de roues du dispositif,
un capteur de rotation de la roue pour déterminer une distance de déplacement de la roue du dispositif, et
au moins un aimant couplé à la roue du dispositif pour activer le capteur de rotation de roue,
le système de commande se réveillant lorsque au moins un frein est désactivé et/ou le dispositif est débranché,
dans lequel, en réponse au réveil du dispositif, le capteur de pivotement de roulette détermine l'orientation angulaire (β) de la roue du dispositif et le capteur de rotation de roue détecte un déplacement de la roue en fonction de la détection de l'au moins un aimant,
dans lequel, en réponse à la réactivation du frein, le système de commande détermine une distance finale de déplacement du dispositif en fonction de l'orientation angulaire (β) détectée de la roue et du déplacement détecté de la roue, et
dans lequel, si le dispositif s'est déplacé par moins d'une distance prédéterminée, le système de commande réassocie automatiquement le dispositif à la même salle.

2. Système de commande selon la revendication 1, dans lequel, si le dispositif s'est déplacé par plus de la distance prédéterminée, le système de commande notifie automatiquement l'utilisateur d'associer une nouvelle salle au dispositif, et dans lequel de préférence la distance prédéterminée est d'un mètre.

3. Système de commande selon la revendication 1 ou 2, dans lequel le capteur de pivotement de roulette détermine l'orientation angulaire (β) de la roue par rapport à une position prédéfinie de la roue.

4. Système de commande selon la revendication 3, dans lequel la roue est fixée à une base qui tourne par rapport à un montant couplé à un cadre du dispositif , dans lequel, dans la position prédéfinie, un axe longitudinal de la base est parallèle à un côté du cadre du dispositif.

5. Système de commande selon la revendication 4, dans lequel un coefficient (Coeff) du capteur de rotation de roue est égal à (π fois le diamètre de la roue)/(un nombre d'aimants sur la roue).

6. Système de commande selon la revendication 5, dans lequel une distance finale (D) de déplacement du dispositif est égale à (X²+Y²)carré, dans lequel X_{n =} Xₙ₋₁(1 + Coeff cos(β)) et Y_{n =} Yₙ₋₁ (1 + Coeff sin(β)), dans lequel X est une distance dans la direction x et Y est une distance dans la direction y.

7. Système de commande selon toute revendication précédente, le système de commande entrant dans un mode de veille si un mouvement de la roue n'est pas détecté pendant une période de temps prédéterminée, et dans lequel de préférence la période de temps prédéterminée est d'une minute.

8. Système de commande selon la revendication 7, le système de commande, après être revenu au mode de veille, se réveillant en réponse à la détection d'un déplacement de la roue par le capteur de rotation de roue.

9. Système de commande selon toute revendication précédente, le système de commande entrant dans un mode de veille après que le dispositif a été automatiquement réassocié à la même salle.

10. Système de commande selon toute revendication précédente, dans lequel le dispositif est réassocié à la salle de l'installation pour connecter le dispositif à un réseau sans fil dans la salle.

11. Système de commande selon toute revendication précédente, dans lequel la roue est fixée à une base qui tourne par rapport à un montant couplé à un cadre du dispositif, dans lequel le capteur de rotation de roulette est fixé à la base.

12. Système de commande selon la revendication 11, dans lequel le capteur de rotation de roue est couplé à la base.

13. Système de commande selon toute revendication précédente, dans lequel l'au moins un aimant comporte une pluralité d'aimants.

14. Système de commande selon la revendication 13, dans lequel les aimants de la pluralité d'aimants sont équidistants autour de la circonférence de la roue.

15. Système de commande selon toute revendication précédente, dans lequel le capteur de pivotement de roulette comporte un accéléromètre et/ou le capteur de rotation de roue comporte un capteur à effet Hall.
